# EUROPEAN PATENT SPECIFICATION

(11) **EP 3 375 367 B1**
(45) Date of publication and mention of the grant of the patent: **19.06.2019**
(21) Application number: 17161518.0
(22) Date of filing: 17.03.2017
(51) Int. Cl.: A61B 5/00, A61B 5/055, G02B 6/02, A61B 1/00, G02B 6/06

(54) **IN VIVO IMAGING DEVICE WITH FIBER OPTIC**
VORRICHTUNG ZUR IN-VIVO-BILDGEBUNG MIT FASEROPTIK
DISPOSITIF D'IMAGERIE IN VIVO AVEC FIBRE OPTIQUE

(43) Date of publication of application: 19.09.2018
(73) Proprietor: Max-Planck-Gesellschaft zur Förderung der Wissenschaften e.V., 80539 München (DE)
(72) Inventor: YU, Xin, 72076 Tübingen (DE); JIANG, Yuanyuan, 72076 Tübingen (DE); RUSSEL, Philip St. J., 91341 Röttenbach (DE); FROSZ, Michael H., 91058 Erlangen (DE); SCHLÜSENER, Jan K., 72076 Tübingen (DE)
(74) Representative: Hannke, Christian

(56) References cited:
- CN-B- 101 612 035
- US-A1- 2002 103 439
- US-A1- 2007 128 749
- US-A1- 2015 185 454
- VAN EIJKELENBORG M A: "imaging with mirostructured polymer fibre", OPTICS EXPRESS, OSA PUBLISHING, vol. 12, no. 2, 26 January 2004 (2004-01-26), pages 342-346, XP002387130, ISSN: 1094-4087, DOI: 10.1364/OPEX.12.000342
- KRISTINA SCHULZ ET AL: "Simultaneous BOLD fMRI and fiber-optic calcium recording in rat neocortex", NATURE METHODS, vol. 9, no. 6, 6 May 2012 (2012-05-06), pages 597-602, XP55406629, ISSN: 1548-7091, DOI: 10.1038/nmeth.2013

## Description

The invention relates to an imaging device to detect electromagnetic radiation, which is preferably fluorescence-emission, wherein a collection unit collects the electromagnetic radiation and transmits it to a sensor device.

State of the art imaging devices are for example described in US2007/0128749A1 and VAN EIJKELENBORG M A: "imaging with mirostructured polymer fibre",OPTICS EXPRESS, OSA PUBLISHING, vol. 12, no. 2, 2004, pages 342-346, XP002387130,ISSN: 1094-4087, DOI: 10.1364/ OPEX.12.000342.

Such an imaging device may be used in connection with functional magnetic resonance imaging (fMRI), which detects brain dynamics based on the neurovascular coupling mechanism¹⁻³. Magnetic resonance imaging (MRI provides strongcontrast between different soft tissues compared to computed tomography (CT). It is especially favorable in neurological (brain), musculoskeletal, cardiovascular, and oncological (cancer) imaging. One crucial application of the MRI is to indirectly map the brain function by examine the hemodynamic response of the vessels, which is linked to neuronal activity. The functional MRI mapping method has mainly been used for brain function and connectivity mapping^{5,6}. Simultaneous fMRI and electrophysiological recordings reveal the neuronal correlate of fMRI signal in both task-related and resting-state conditions^{7,8}. However, the detailed cellular contributions to the fMRI signal remain unclear.

One of the most exciting directions of molecular imaging is the development of genetically encoded indicators to sense Ca²⁺ and membrane voltage signals. The high specificity and fast kinetics of the endogenous indicators makes it possible to monitor the dynamic signal from specific cell types in transgenic animals or in specific functional nuclei by viral vector expression in the animal brain. With conventional confocal and multiphoton microscopy, the neural activity can be directly imaged from individual cells based on these indicators, but the optical penetration depth is restricted to less than 1 mm given the absorption of visual light by tissues⁹. Fiber-optical recordings have been implemented to detect brain dynamic signals in animals with fMRI, because of its ability to record deep brain activity without electromagnetic interference in the MRI-scanner in both anesthetized and alert animals. However, it remains challenging to acquire the dynamic signal from single cells with an ultrafast sampling rate.

Previously, a large number of voxels of the calcium transient from both neurons and glia cells through a single fiber combined with fMRI was able to predict the blood-oxygen-level-dependent (BOLD) responses from fluorescence recording¹². With a standard single core optical fiber, light was guided by total internal reflection. The bulk fluorescent signals may reflect activity of a large population of cells labelled specifically by calcium sensitive dyes.. To achieve simultaneously fast measurement of fluorescent signals from multiple cell populations or multiple sites across the brain in a large field of view (FOV), several techniques have been proposed. For example, a head-mounted microscope and spatially selective fiber bundles that permit cellular or near-cellular resolution in rodents have been proposed^{13,14}. However, these solutions remain low sampling rate, which do not provide the feasiblity for fast sampling of the neuronal activity with sufficient signal-to-noise ratio. Recently, an alternative method for genetically defined activity is a combination of multiple fibers and a sCMOS (scientific complementary metal-oxide semiconductor) camera to achieve concurrent acquisition¹⁵ of calcium signal activity from neuronal populations at different brain regions, but is not able to detect signals at the cellular level.

The object of the invention is therefore to provide an imaging device that overcomes the above-mentioned disadvantages. In particular the imaging device should allow an *in vivo* dynamic signal of individual cells with ultra fast sampling rate and sufficient signal-to-noise ratio (SNR).

The problem is addressed by an imaging device to detect electromagnetic radiation, which is preferably fluorescence-emission, wherein a collection unit collects the electromagnetic radiation and transmits it to a sensor device, wherein the sensor device is a photomultiplier array, preferably a silicon photomultiplier array, and the collection unit comprises a multichannel optical waveguide in the form of a microstructured fiber, comprising at least two solid cores separated by at least one hollow tube, wherein each solid core propagates a portion of the collected electromagnetic radiation and wherein the emitted portion of electromagnetic radiation of each individual solid core is projected by a projection/ magnification unit on an individual pixel of the photomultiplier array.

The electromagnetic radiation in question is in the form of an optical signal and is preferably in the range of 400 nm to 1000 nm. The imaging device comprises a multichannel optical waveguide in the form of a microstructured fiber. Conventional optical fibers use the effect of total internal reflection to guide the elctromagnetic radiation. The guiding occurs within a core of refractive index higher than refractive index of the surrounding material. The index change is obtained through different doping of the core and the cladding or through the use of different materials. In contrast to conventional microstructured fibers, the guiding is obtained through manipulation of waveguide structure. Typically, a microstructured fiber comprises a solid core which is surrounded by air holes which go through the whole length of the fiber, wherein the guiding of the electromagnetic radiation is obtained through the presence of theses air holes in the area surrounding the solid core. The guiding can be based on two principles. It can be based on the effect of total internal reflection (Index guided fibers), where the region with the missing hole has a higher refractive index, similar to the core of a conventional fiber. Alternatively, the guiding can be based on the photonic bandgap effect, in which the guiding is obtained through constructive interference of scattered light. Typically, the expression photonic crystal fibers (PCF) is used for fibers faciliating the photonic bandgap effect. As such photonic crystal fibers may be considered as a subgroup of microstructerd optical fibers (MOF). The use of PCFs/MOFs greatly expands the capabilities of optical waveguides with extraordinary properties of the optical waveguide such as low light loss, high efficiency, small nonlinearities, high beam quality and scalability¹⁶⁻¹⁸.

The imaging device at hand comprises at least two solid cores separated by at least one hollow tube. The collected electromagentic radiation is transmitted through the solid cores. The hollow tubes reduce cross-talk issues between neighboring channels. The utilization of such a microstructure optical fiber bears therefore the advantage of high signal-to-noise ratio. Furthermore, such fiber may be fabricated with an individual core in the a few micronrange, which allows to detect signals of an individual cell.

The imaging device comprises further a sensor device in the form of a photomultiplier array. Preferably, a silicon photomultiplier array is utilized. However, photomultiplier devices based on different materials or structures may be utilized, providing these photomultipiers exhibit sufficient key performance parameters, such as gain, response time and spectral range. In contrast to sCMOS- or charge-coupled device (CCD)-devices, photomultipliers do not store charge and respond to changes in input light fluxes within a few nanoseconds, they can therefore be used for the detection and recording of extremely fast events. Typically, photomultipliers generate low noise values and darkcurrents. This results in a large dynamic range over which the electrical current output accurately reflects the photonflux. Further, photomultipliers provide a comparatively large gain.

Recent studies either detect the single fiber optical signal with a high sampling rate through a silicon-based photodiode up to 1 kHz^{12,19} or acquire sCMOS-based images with 10-30 Hz¹³⁻¹⁵. The sCMOS sensor applies a line-scanning mapping scheme to acquire the optical signal with high spatial resolution through individual lines of a micro-scale photodiode. Given the size of field of view (FOV) and exposure time, its sampling rate varies from tens to hundreds of Hz. Given the performance metrics of the photodiodes used in the sCMOS camera²⁰, it is challenging to detect the in vivo dynamic signal of individual cells with an ultrafast sampling rate (from 200 Hz to >1 kHz) and sufficient signal-to-noise ratio using the conventional line-scanning method. The following comparison shows the key performance metrics of a silicon photomulitplier (SiPM), a sCMOS photodiode and a CCD camera:

| Device Type | Gain | Response Time (ns) | Spectral Range (nm) |
|---|---|---|---|
| sCMOS | 1 or less | 150-200 | 190 - 1100 |
| CCD | 1 or less | 2 x 10⁶ - 1.4 x 10⁸ | 200 - 1200 |
| SiPM | 10⁶ | >0.003 | 400 -1000 |

Thus, by the utilization of a photomultiplier array, in particular a silicon photomultiplier array, a sampling rate of > 1kHz can be achieved. Due to the projection of the emitted portion of electromagnetic radiation of each individual solid core on an individual pixel or cell of the photomultiplier array, an efficient detection without unneccesarry loss of elecotrmagnetic radiation is ensured. Accordingly, an ultrafast sampling rate with single-cell specificity is provided by the imaging device of the present invention.

According to a preferred example, the multichannel optical waveguide comprises a collection-end-portion, which collects the electromagnetic radiation, a main portion and an emission-end-portion, where the portions of electromagnetic radiation of each solid core are emitted. Advantageously the solid cores are arranged in a two dimensional n x m array, n and m being natural numbers. Preferably the solid cores are in each dimension separated by each other by at least one hollow tube. Advantageously, each solid core is surrounded by at least four hollow tubes, preferably each solid core is surrounded by six hollow tubes. Preferably the multichannel optical waveguide has an output in the form of an n x m beam array, n and m being natural numbers. Preferably the portions of the collected electromagnetic radiation propagate from collection-end-portion in the solid cores of the n x m array to the emission-end-portion, where the portions of electromagnetic radiation are emitted. Due to the two dimensional n x m array arrangement of the solid cores, a beam array comprising those portions is emitted. Preferably, the beam array may be a 4 x 4-, an 8 x 8- or a 16 x 16-array. Since each of the beams is projected by a projection-/ magnification unit on an individual pixel of the photomultiplier array, the photomultiplier array has at least the same dimensions as the beam array. Thus, 16, 64 or 144 multichannel (dynamic) signals may be acquired in parallel.

According to a preferred example, the solid cores and the hollow tubes consist of a glass material, preferably a silica glass or a polymeric glass or a chalcogenide glass. Preferably the solid cores have a diameter in the range of 5 µm to 50 µm, more preferably in the range of 8 µm to 10 µm. Advantageously, the hollow tubes have a diameter in the range of 5 µm to 100 µm preferably in the range of 8 µm to 10 µm. Advantageously, the diameter of the multi-channel optical waveguide is in the range of 20 µm to 3-4 mm, preferably in the range between 50 µm to 300 µm, more preferably in the range between 200 µm to 250 µm. A multichannel optical waveguide comprising such dimensions in the submillimeter or micrometer range ensures a large enough penetration depth for the application in connection with the fMRI method. Further single-cell specificity is obtained.

According to a further prefered example, the collection-end-portion of the multichannel optical waveguide comprises hollow tubes which have a larger diameter than the hollow tubes in the main portion of the multichannel optical waveguide and the hollow tubes in the emission-end-portion of the multichannel optical waveguide. Preferably, the hollow tubes with a larger diameter are stacked on the hollow tubes with a smaller diameter in a transition region between the collection-end-portion and the main portion. By such an arrangement, the capillarry effect of the hollow tubes in the main portion of the multichannel optical waveguide may be suppressed. Preferably the main optical signal is deliverd through the solid cores, weherby the potential signal loss through the hollow cores is reduced.

According to a further preferred example, the projection-/ magnification unit comprises a first magnification unit and a second magnification unit. Preferably both magnification units comprise an infinity-corrected optical system in the form of an objective and a tube lens. Preferably, each beam of the beam array is magnified to about the size of a pixel of the photomultiplier array. Preferably, a pixel of the photomultiplier array has a size in the range of 1 mm² to 10 mm², more preferably it has a size of 3 mm². Preferably, the collected electromagnetic radiation is in a field of view at a few microns. Thus, each beam of the beam array, which has preferably a beam size in the micrometer-range, is magnified to a size in the millimeter-range. It is further advantageous that the emission-end-portion of the multichannel optical waveguide is located exactly in the front focal plane of the objective lens, which transmits the individual beams emitted from the emission-end-portion in an infinite distance. Thus, the optical path behind the objective is parallel. Preferably, a sub-micron resolution launch stage system (X, Y, Z axis) may be used for adjusting the emission-end-portion of the multichannel optical waveguide accordingly. Preferably, optical elements such as a fluorescence filter and/or a dichroic mirror may be arranged in the optical path after the objective. Since parallel beams are only displaced by plane-parallel optical elements the optical elements can therefore be displaced without distorting the beam path. The tube lens is arranged in the optical path after the objective. This tube lens is used to compensate for residual image aberration.

As the required beam magnification is preferably about 200 times, which is not feasible with one objective lens, a second magnification unit is arranged in the optical path after the first magnification unit, which also comprises an objective and a tube lens. The focal plane of this second objective lens is advantageously located in the primary image plane that is the image plane of the tube lens of the first magnification unit. The photomultiplier array is then preferably in the secondary image plane, which is the image plane of the tube lens for aberration correction of the second magnification unit.

According to a further preferred example, the projection/magnification unit is able to direct a portion of each beam in the beam array to an optical detector. Preferably the optical detector is able to detect the spatial orientation of the beam array and is able to provide data regarding spatial orientation of the beam array. Preferably, the projection/magnification unit comprises a beam splitter, by which designated portion of each beam in the beam array is directed to the according optical detector. Advantageously, the beam splitter is arranged in the optical path between the first magnification unit and the second magnification unit. Preferably, the beam splitter is arranged in the primary image plane. Advantageously, the beam splitter is a 50:50 beam splitter.

Preferably, the optical detector is a scientific complementary metal-oxide-semiconductor (sCMOS) camera.

In a further preferred example, the imaging device comprises a control unit, which is able to receive data regarding the spatial orientation of the beam array from the optical detector. Preferably, the control unit is further able to control a multi-axis motorized stage on which the sensor device in the form of the photomultiplier array is arranged. Preferably the multi-axis motorized stage is adjustable along the translational directions (X, Y, Z) determined by a Cartesian coordinate system and a tilting angle (θ) of the array surface plane. Preferably, the control unit also controls the sub-micron resolution launch stage system (X, Y, Z axis) for adjusting the end-portion of the multichannel optical waveguide. Advantageously, the control unit is also able to receive the data from the sensor device.

It is advantageous that the control unit is able to process the data regarding spatial orientation of the beam array, which is preferably a sCMOS-image, recorded from the primary image plane and to adjust the orientation of the photomultiplier array by controlling the multi-axis motorized stage in real-time.

In a further preferred example the control unit is able to process the data regarding the spatial orientation of the beam array, which is preferably a sCMOS-image, recorded from the primary image plane and the data from the sensor device and is able to adjust the orientation the photomultiplier array by controlling the multi-axis motorized stage in real-time. Advantageously, the control unit extracts the translation and rotation of each beam of the beam array and derives a transformation matrix (translation and rotation matrix) from this information.

Preferably, the beam array scaling factor is determined by the magnification in the just beforehand determined Z axis direction. It is further preferred that the beam array illumination patterns will first be imaged on the optical detector as multichannel beam validation. A control unit calculates the transformation matrix which verifies the feasibility of an appropriate alignment. It is advantageous that the detected dynamic signal from both the optical detector and the sensor device are processed in real-time to adjust the appropriate alignment and verify the single-cell specificity from certain channels when the collection-end-portion of the multichannel optical waveguide is inserted into the animal brain. The expected outcome of this design is to establish a functional optical recording device with single-cell specificity.

According to a further preferred example, the imaging device comprises a light source, which is coupled in the multichannel optical waveguide and provides the excitation energy preferably for the fluorescence emission. Advantageously, the light source is a coherent light source such as a laser. However other light sources such as LED might also be conceivable.

Preferably the excitation intensity is controlled by the controlling unit. Advantageously, the excitation is controlled based on detected photobleaching effect in the data provided by the sensor device and/or data provided by the optical detector. Such indications are for example altered signal-to-noise ratios. Photobleaching is the photochemical alteration of a fluorophore molecule such that it permanently loses fluoresce. The excitation control may for example comprise reducing the excitation intensity, reducing the excitation frequency or the time-span of exposure.

The object of the disclosure is also addressed by an imaging system comprising an imaging device according to one of the previous embodiments and a MRI-scanner.

Advantageously, a single-vessel fMRI measurement can be established by such an imaging system. This fMRI measurement allows a detection of the hemodynamic fMRI signal from individual arteriole and venules in the cortex far beyond the penetration depth of conventional optical methods in animal brains. Such an imaging system makes it possible to identify the signaling mechanism of the neuron-glia-vascular (NGV) circuit in the brain. An optical imaging method based on the beam array of a multichannel optical waveguide may be implemented with single-vessel fMRI to identify dynamic signaling from single cells and vessels in rodents or other mammals. Advantageously, an fMRI signal from individual arterioles and venules with optogenetics may be attained in both cortical and subcortical regions. Furthermore, the region-specific NGV coupling events may be mapped with such an image system. It is possible to establish simultaneous fMRI with single fiber-based Ca²⁺ recording. A combination of the MOF array-mediated optical recording with simultaneous single vessel fMRI, allows to directly map the signaling propagation in the NGV circuit at different brain regions.

The object of the disclosure is also addressed by a method for analyzing a sample preferably on the basis of fluorescence signals, using an imaging device according to any one of the preceding embodiments comprising:
- collecting the fluorescence signal with a collection unit comprising a multichannel optical waveguide in the form of a microstructured fiber, comprising at least two solid cores separated by at least one hollow tube, wherein in each solid core propagates a portion of the collected electromagnetic radiation;
- emitting the portion of electromagnetic radiation of each individual solid core in the form of a beam array;
- projecting the beam array on an individual pixel of a sensor device in the form of a photomultiplier array by a projection-/ magnification unit.

The method may comprise the same examples and advantages as described in conjunction with the above-described imaging device.

According to a further preferred example, each beam of the beam array is magnified by the projection/magnification unit to about the size of an individual pixel of the sensor array.

According to a further preferred example, a portion of the beam array is projected by the projection/magnification unit on an optical detector, which is able to detect the spatial orientation of the beam array. Preferably the orientation of the sensor device is adjusted in real-time according to data regarding the spatial orientation of the beam array and/or the data from the sensor device. Hereby an optimal illumination of the individual pixels of the sensor device is ensured.

By transmitting the beam array through the described microstructured fiber and by projecting the beam array on the sensor device and the optical detector, an ultrafast sampling rate and a single-cell specificity is acquired.

According to a further preferred example, a fluorescence signal is generated in the sample by providing excitation energy with a light source. Preferably, the excitation is controlled based on detected indications on photobleaching in the data provided by the sensor device and / or data provided by the optical detector.

According to a further preferred example, the fluorescence signals are combined with real-time fMRI, wherein an excitation pattern, for example frequency, duration and amplitude, can be correlated to a MRI sequence design.

Further advantages, aims and properties of the present disclosure will be described by the appended drawings and the following description.

In the drawings:
- Fig. 1: shows the imaging device according to one example;
- Fig. 2: shows the imaging device according to one example;
- Fig. 3: shows the multichannel optical waveguide according to one example;
- Fig. 4: shows the multichannel optical waveguide according to one example and the sensor device according to one example;
- Fig. 5: shows the adjustment of the sensor device according to the orientation of the beam array;
- Fig. 6: shows the sensor device according to one example;
- Fig. 7: shows a block diagram of the imaging device according to one example;
- Fig. 8: shows a MRI-system;
- Fig. 9: shows a "single unit" mapping scheme on the neuron-glia-vascular circuit with fMRI and MOF array-based optical imaging;

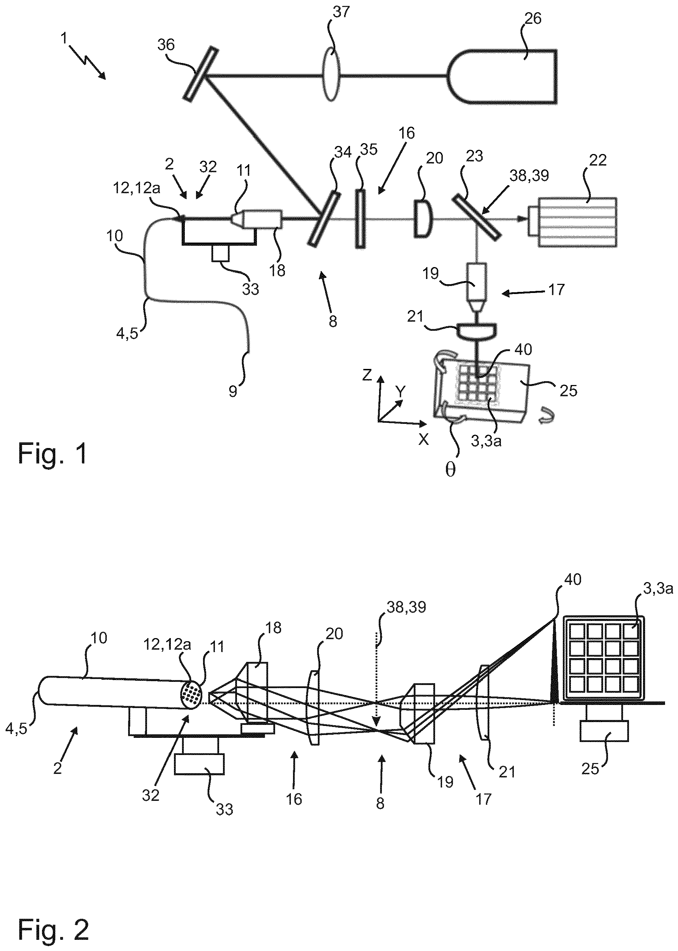

In Fig.1 and Fig. 2, an imaging device (1) to detect electromagnetic radiation, which is preferably fluorescence-emission is depicted. The imaging device (1) comprises a collection unit (2), which collects the electromagnetic radiation and transmits it to a sensor device (3), wherein the sensor device (3) is a photomultiplier array (3a), preferably a silicon photomultiplier array, and the collection unit (2) comprises a multichannel optical waveguide (4) in the form of a microstructured fiber (5), comprising at least two solid cores (6) separated by at least one hollow tube (7), wherein in each solid core (6) propagates a portion of the collected electro-magnetic radiation and wherein the emitted portion of electro-magnetic radiation of each individual solid core (6) is projected by a projection-/ magnification unit (8) on an individual pixel (3b) of the photomultiplier array (3a).

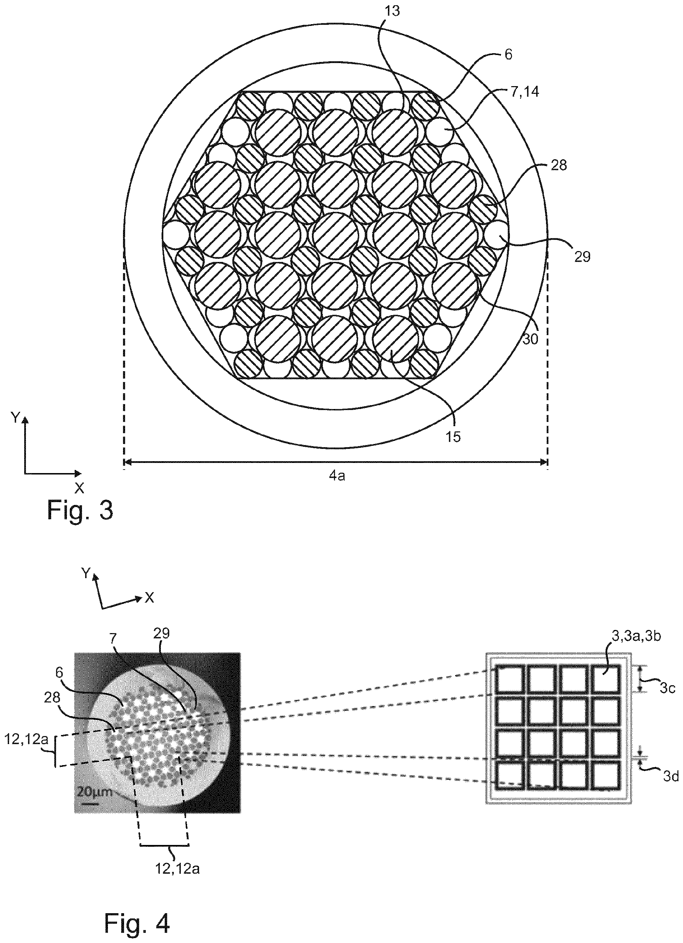

The multichannel optical waveguide (4) comprises a collection-end-portion (9), which collects the electromagnetic radiation, a main portion (10) and an emission-end-portion (11), where the portions of electromagnetic radiation of each solid core (6) are emitted. The solid cores (6) are arranged in a two dimensional n x m array (12), n and m being natural numbers. The solid cores (6) are in each dimension separated by each other by at least one hollow tube (7), wherein the multichannel optical waveguide (4) has an output in the form of an n x m beam array (12a). Fig. 3 shows the collection-end-portion (9) of the multichannel optical waveguide (4) and Fig. 4 shows the emission-end-portion (11).

The light is transmitted through the solid-core array (12). The solid cores (6) are interleaved by the hollow tubes (7). Here, the size of each solid core (6) is designed to be 8-10 µm diameter, which is close to the size of neuronal soma^{21,22}. The solid cores (6) are separated by the hollow tubes (7), which have a diameter of 8-10 µm and serve to reduce the cross-talk of optical signal through the solid cores (6). The multichannel optical waveguide (4) has a diameter in the range of 200-250 µm.

As depicted in Fig. 3 and Fig. 4 the solid cores (6) are arranged in horizontal lines (28), which extend along a horizontal direction X. In such a horizontal line (28) a solid core (6) at the position n is separated from its neighboring solid core (6) at the position n+1 by a hollow tube (7). The separating hollow tube (7) abuts the two neighboring solid cores (6). A horizontal line (28) of solid cores (6) is separated by a horizontal line (29) of hollow tubes (7) in a vertical direction Y. The solid cores (6) and the hollow tubes (7) are arranged in a highest-density lattice arrangement, which is the hexagonal packing arrangement. The centers of the solid cores (6) and the hollow tubes (7) are arranged in a hexagonal lattice (staggered rows). Hence, each solid core (6) is surrounded by 6 hollow tubes (7). This unique design merges the advantage of the PCF/MOF fiber design flexibility and fiber bundle imaging scheme. To optimize the spatial detection of single cell dynamic signals, different array patterns with varied sizes of solid and hollow tubes are conceivable.

The collection-end-portion (9) of the multichannel optical waveguide (4) comprises hollow tubes (13), which might have a larger diameter than the hollow tubes (14) in the main portion (10) of the multichannel optical waveguide (4) and the hollow tubes (15) in the emission-end-portion (11) of the multichannel optical waveguide (4). In a transition region (30) between the collection-end-portion (9) and the main portion (10) of the optical multichannel waveguide (4), the hollow tubes (13) with a larger diameter are stacked on the hollow tubes (14) with a smaller diameter. Hereby a hollow tube (13) with a larger diameter is stacked on four neighboring hollow tubes (14) with a smaller diameter. By such an arrangement the capillary effect of the hollow tubes (7) in the main portion of the multichannel optical waveguide (4) is suppressed.

Fig. 4 shows the projection of the n x m beam array (12a) stemming from the n x m array (12) of the solid cores (6). Here a 4 x 4 array is depicted. The dashed lines show the projection from solid core (6) at array-position a_{1,1} and the projection from solid core (6) at array-position a_{4,4}. These beams are projected on a 4 x 4 photomultiplier array with 16 pixels. The beam stemming from solid core (6) at array-position a_{1,1} is projected to pixel a_{1,1} in the photomultiplier array. The beam stemming from solid core (6) at array-position a_{4,4} is projected to pixel a_{4,4} in the photomultiplier array. The depicted 4 x 4 beam array (12a) and photomultiplier array (3a) are limited to 4 x 4 for reasons of simplicity. Typically, an 8 x 8 or a 12 x 12 photomultiplier-/ beam array (3a, 12a) is utilized to acquire 64 and 144 multichannel dynamic signals in parallel. The pixel size (3c) of an individual pixel (3b) of the photomultiplier array (3a) is about 3 mm. The distance (3d) between two individual pixels (3b) photomultiplier array (3a) is about 0.2 mm.

The projection-/ magnification unit (8) comprises a first magnification unit (16) and a second magnification unit (17): The first magnification unit (16) comprises an infinity corrected optical system in the form of a first objective (18) and a first tube lens (20). The second magnification unit (17) comprises an infinity corrected optical system in the form of a second objective (19) and a second tube lens (21). Both tube lenses (20, 21) are used to compensate for residual image aberration. Each beam of the beam array (12a), which has preferably a beam size in the micrometer-range, is magnified to a size in the millimeter-range to ensure an optimal illumination of each individual pixel (3b) of the photomultiplier array (3a).

The emission-end-portion (11) of the multichannel optical waveguide (4) is located exactly in the front focal plane (32) of the first objective (18) to ensure a transmission of the individual beams of the beam array (12a) in an infinite distance. Thus, the optical path behind the first objective (18) is parallel. A sub-micron resolution launch stage system (33) may be used for adjusting the emission-end-portion (11) of the multichannel optical waveguide (4) to ensure an exact positioning of the emission-end-portion (11) in the front focal plane (32) of the first objective lens (18). In the optical path behind the first objective (18) are a dichroic mirror (34) an emission filter (35) and the first tube lens (20), which is used to compensate for residual image aberration, arranged. Parallel beams are only displaced by plane-parallel optical elements. The optical elements can therefore be displaced without distorting the beam path.

The dichroic mirror (34) allows the emitted beam array (12a) to pass towards the emission filter (35) or the first tube lens (20). Further, the dichroic mirror (34) couples the excitation light from the light source (26) into the first objective (18). The light source (26) is in this case a laser. Due to the arrangement of the emission-end-portion (11) of the multichannel optical waveguide (4) in the front focal plane (32) of the first objective (18), the excitation light is optimally focused into the multichannel optical waveguide (4). The laser emission may be adjusted in terms of wavelength and / or intensity by a filter wheel (37). Further, a mirror (36) is utilized to guide the laser light.

As the required beam magnification is preferably about 200 times, which is not feasible with one objective lens, a second magnification unit (17) is arranged in the optical path after the first magnification unit (16). The focal plane (38) of this second objective lens (19) is located in the primary image plane (39) that is the image plane of the first tube lens (20). The secondary tube lens (21) is arranged in the optical path after the secondary objective (19). The photomultiplier array (3a) is located in the secondary image plane (40) that is the image plane of the second tube lens (21).

In the primary image plane (39) a beam splitter (23) is arranged. A portion of each beam of the beam array (12a) is therefore directed to the photomultiplier array (3a) via the second magnification unit (17). Another portion of each beam of the beam array (12a) is directed to an optical detector (22), which is able to detect the spatial orientation of the beam array (12a) and is able to provide information regarding spatial orientation of the beam array (12a).

The imaging device (1) comprises a control unit (24), which is able to receive data regarding the spatial orientation of the beam array from the optical detector (22) and is able to control a multi axis motorized stage (25) on which the photomultiplier array (3a) is arranged. The control unit (24) is able to process the data regarding spatial orientation of the beam array (12a) and the data from the sensor device (3) and is able to adjust the orientation the photomultiplier array (3a) by controlling the multi axis motorized stage (25) in real-time.

The multi-axis motorized stage (25) is adjustable along the translational directions (x, y, z) determined by a Cartesian coordinate system and a tilting angle (θ) of the array surface plane. The control unit (24) may also control the sub-micron resolution launch stage system (33) for adjusting the emission-end-portion (11) of the multichannel optical waveguide (4). Further, the control unit (24) is also able to receive the data from the sensor device.

The optical detector (22) records images of the primary image plane (39). From this data the orientation of the beam array (12a) is evaluated by the control unit (24). Based on this evaluation, a transformation matrix (43) is derived by the control unit (24). This transformation matrix (43) is utilized to control the multi-axis (x, y, z, *θ*) motorized stage (25) to adjust the sensor array in real-time. The spatial orientation of the beam array (12a) matches the orientation of the photomultiplier array (3a). Moreover, a favourable perpendicular impact of the beams may be achieved. Thus, an optimal illumination of the photomultiplier array (3a) is achieved by the projection-/ magnification unit (8), since each individual beam of the beam array (12a) is magnified to the size of the photomultiplier array (3a) and at the same time the spatial orientation of the photomultiplier array (3a) is matched to the spatial orientation of the beam array (12a).

In the present case, the optical detector (22) is a scientific complementary metal-oxide-semiconductor (sCMOS) camera and the photomultiplier is a silicon photomultiplier (SiPM).

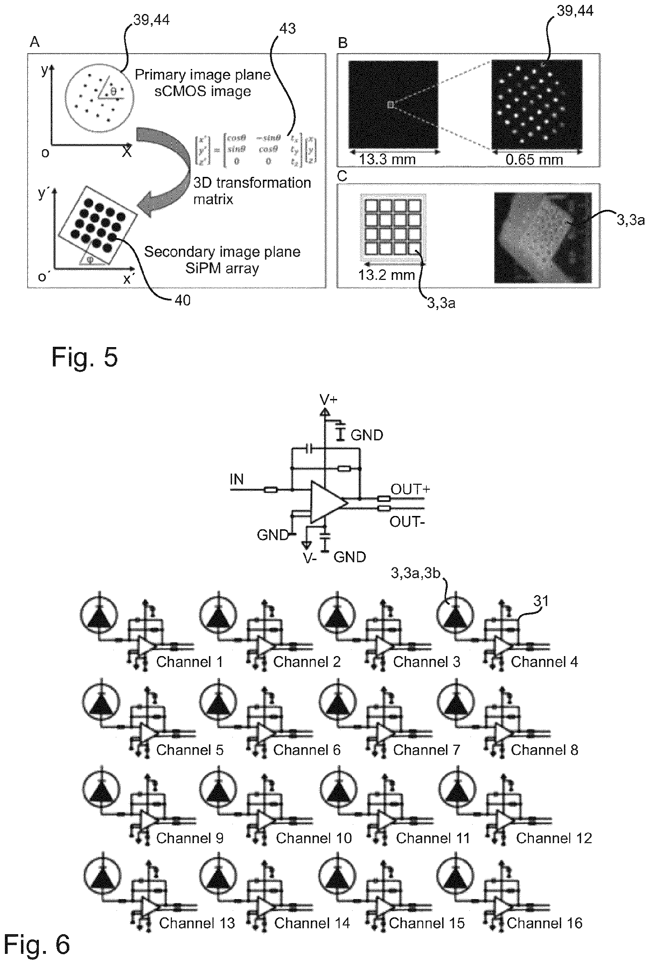

The sensor device comprises further a preamplifier (31) which is shown in Fig. 6. An exemplary 4 x 4 photomultiplier array is depicted, wherein each pixel is provided with an individual preamplifier (31). Further, Fig. 6 shows an enlarged circuit with an operational amplifier as example for a preamplifier circuit.

Fig. 5 depicts the principle of simultaneous sCMOS camera (22) and SiPM (3a) measurements. Fig. 5A shows the transformation matrix (translation and rotation matrix multiplication) (43) from the sCMOS image (44) to the SiPM array (3a) for SiPM array (3a) positioning. An example of images acquired from sCMOS camera in the different FOV is shown in Fig. 5B. In the left panel of Fig. 5B, the whole FOV (field of view) of the sCMOS image (2048x2048 pixels) is depicted. In the right panel of Fig. 5B a sub-array FOV (100x100 pixels) with fast image acquisition with low magnification objective is depicted. With small FOV (100x100 pixels, each pixel size is 6.5 µm), the sCMOS images can be recorded up to ∼ 1k frames/s. This small FOV is able to directly image the multi-channel beams from the beam array (12a). Fig. 5C displays an example of LED light illumination through the solid core beam array (12) precisely projection to the same size of the SiPM array (3a).

The above-described imaging device (1) may be used in connection with an imaging system (27). This imaging system (27) comprises said imaging device (1) and a MRI-scanner (45). The imaging system (27) may be utilized to combine the fluorescence signals with real-time fMRI, wherein an excitation pattern, for example frequency, duration and amplitude, can be correlated to a MRI sequence design.

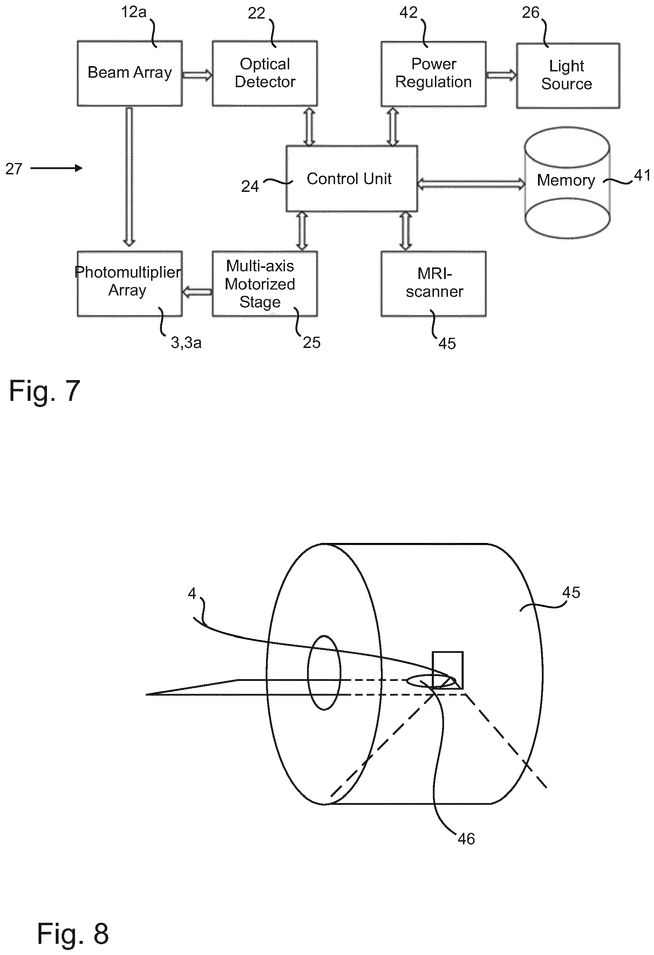

Fig. 8 depicts a MRI-scanner (45) in principle. Such a MRI-scanner (45) uses a usually passive magnetic field preferably in the range of ca. 3 T to ca. 21 T, more preferably in the range of ca. 7 T to ca. 14 T, and a bore diameter of preferably in the range of 6 cm to 50 cm, more preferably 12 cm. In the bore of the MRI-scanner (45) the subject, for example a rodent like rat, is positioned. The multichannel optical waveguide (4) in the form of a microstructured fiber (5) is inserted in the brain of a subject (46).

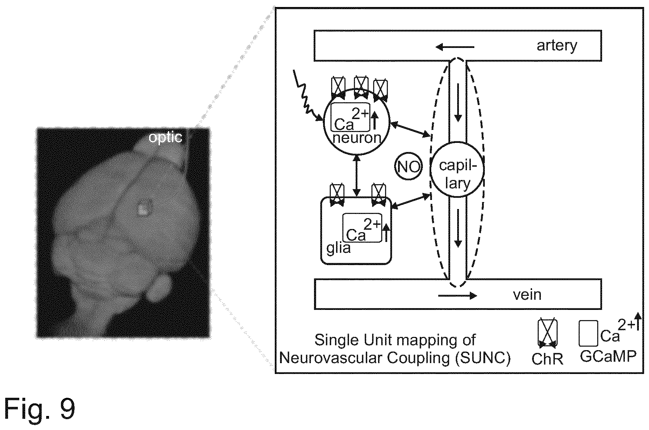

The imaging system (27) is designed for animal brain imaging, which can be used on anesthetized and alert animals. The neuroscientific objective of the imaging system (27) in the form of multi-modal fMRI platform is described in Fig. 9. Anesthetized or alert animals (46) will be positioned inside the MRI-scanner (45). The multichannel optical waveguide (4) in the form of a microstructured fiber (5) will be inserted to target the brain regions expressing genetically encoded indicators (e.g. Ca²⁺ indicator, GCaMP6f) in both neurons and astrocytes. Channelrhodopsin-2 (ChR2) could be co-expressed with genetically encoded indicators for optogenetic activation in brain regions, which may not be easily evoked by a task related block design paradigm. Also, the optogenetically activated single-vessel fMRI signal could be directly mapped in the deep layer cortex. The imaging system (27) makes it possible to directly map the dynamic signal propagation through the neuron-glia-vascular (NGV) network at the single cell and single vessel level, simultaneously.

Fig. 7 displays the imaging system (27) and the functional relation between the control unit (24), the beam array (12a), the optical detector (22), the multi-axis motorized stage (25) the MRI-scanner (45) and the light source (26).

The beam array (12a) illumination patterns will first be imaged on the sCMOS camera (22) as multichannel beam validation. The control unit (24) will calculate the transformation matrix (43) based on the sCMOS camera image (44). The transformation matrix (43) verifies the feasibility of an appropriate alignment of the SiPM array (3a) and is utilized to control the multi-axis (x, y, z, *θ*) motorized stage (25) to adjust the sensor array in real-time. The detected dynamic signal from both sCMOS (22) and SiPM array (3, 3a) will be processed in real-time to adjust the appropriate alignment and verify the single-cell specificity from certain channels when the collection is inserted into the animal brain. The expected outcome of this design is to establish a functional beam array based optical recording device with single-cell specificity.

The control unit (24) is further connected to a power regulation device (42) for the light source (26). Hereby the excitation may be controlled based on detected indications on photo-bleaching in the data provided by the sensor device (3) and / or data provided by the optical detector (22).

Finally, the control unit (24) is connected with a memory device (41).

The invention is defined in the appended claims.

### List of reference numerals

- 1: imaging device
- 2: collection unit
- 3: sensor device
- 3a: photomultiplier array
- 3b: individual pixel of the photomultiplier array
- 3c: pixel size of an individual pixel
- 3d: distance between two individual pixels
- 4: multichannel optical waveguide
- 4a: diameter of multichannel optical waveguide
- 5: microstructured fiber / photonic crystal fiber
- 6: solid core
- 7: hollow tube
- 8: projection-/ magnification unit
- 9: collection-end-portion of the multichannel optical waveguide
- 10: main portion of the multichannel optical waveguide
- 11: emission-end-portion of the multichannel optical waveguide
- 12: two dimensional n x m array of solid cores
- 12a: two dimensional n x m beam array
- 13: hollow tube in collection-end-portion of the multichannel optical waveguide
- 14: hollow tube in main-portion of the multichannel optical waveguide
- 15: hollow tube in emission-end-portion of the multichannel optical waveguide
- 16: first magnification unit
- 17: second magnification unit
- 18: first objective
- 19: second objective
- 20: first tube lens
- 21: second tube lens
- 22: optical detector
- 23: beam splitter
- 24: control unit
- 25: multi-axis motorized stage
- 26: light source
- 27: imaging system combined with a MRI-scanner
- 28: horizontal line of solid cores
- 29: horizontal line of hollow tubes
- 30: transition region between the collection-end-portion
- 31: photomultiplier preamplifier
- 32: front focal plane of the first objective lens
- 33: launch stage system
- 34: dichroic mirror
- 35: emission filter
- 36: mirror
- 37: filter wheel
- 38: focal plane of this second objective lens
- 39: primary image plane
- 40: secondary image plane
- 41: memory device
- 42: power regulation device
- 43: the transformation matrix
- 44: sCMOS image
- 45: MRI-scanner
- 46: subject
- Θ: angle
X-axis
Y-axis
x-axis
y-axis
z-axis

### List of citations

1 Bandettini, P. A., Wong, E. C., Hinks, R. S., Tikofsky, R. S. & Hyde, J. S. Time Course Epi of Human Brain-Function during Task Activation. Magnet Reson Med 25, 390-397, doi:DOI 10.1002/mrm.1910250220 (1992).
2 Kwong, K. K. et al. Dynamic magnetic resonance imaging of human brain activity during primary sensory stimulation. Proc Natl Acad Sci USA 89, 5675-5679 (1992).
3 Ogawa, S. et al. Intrinsic Signal Changes Accompanying Sensory Stimulation - Functional Brain Mapping with Magnetic-Resonance-Imaging. P Natl Acad Sci USA 89, 5951-5955, doi:DOI 10.1073/pnas.89.13.5951 (1992).
4 Ogawa, S., Lee, T. M., Kay, A. R. & Tank, D. W. Brain Magnetic-Resonance-Imaging with Contrast Dependent on Blood Oxygenation. P Natl Acad Sci USA 87, 9868-9872, doi:DOI 10.1073/pnas.87.24.9868 (1990).
5 Biswal, B., Yetkin, F. Z., Haughton, V. M. & Hyde, J. S. Functional Connectivity in the Motor Cortex of Resting Human Brain Using Echo-Planar Mri. Magnet Reson Med 34, 537-541, doi:DOI 10.1002/mrm.1910340409 (1995).
6 Fox, M. D. & Raichle, M. E. Spontaneous fluctuations in brain activity observed with functional magnetic resonance imaging. Nature Reviews Neuroscience 8, 700-711, doi:10.1038/nrn2201 (2007).
7 Logothetis, N. K., Pauls, J., Augath, M., Trinath, T. & Oeltermann, A. Neurophysiological investigation of the basis of the fMRI signal. Nature 412, 150-157, doi:Doi 10.1038/35084005 (2001).
8 Scholvinck, M. L., Maier, A., Ye, F. Q., Duyn, J. H. & Leopold, D. A. Neural basis of global resting-state fMRI activity. P Natl Acad Sci USA 107, 10238-10243, doi:10.1073/pnas.0913110107 (2010).
9 Ntziachristos, V. Going deeper than microscopy: the optical imaging frontier in biology. Nat Methods 7, 603-614, doi:10.1038/nmeth.1483 (2010).
10 Lee, J. H. et al. Global and local fMRI signals driven by neurons defined optogenetically by type and wiring. Nature 465, 788-792, doi:10.1038/nature09108 (2010).
11 Gunaydin, L. A. et al. Natural neural projection dynamics underlying social behavior. Cell 157, 1535-1551, doi:10.1016/j.cell.2014.05.017 (2014).
12 Schulz, K. et al. Simultaneous BOLD fMRI and fiber-optic calcium recording in rat neocortex. NatMethods 9, 597-602, doi:10.1038/nmeth.2013 (2012).
13 Ghosh, K. K. et al. Miniaturized integration of a fluorescence microscope. Nat Methods 8, 871-878, doi:10.1038/nmeth.1694 (2011).
14 Szabo, V., Ventalon, C., De Sars, V., Bradley, J. & Emiliani, V. Spatially selective holographic photoactivation and functional fluorescence imaging in freely behaving mice with a fiberscope. Neuron 84, 1157-1169, doi:10.1016/j.neuron.2014.11.005 (2014).
15 Kim, C. K. et al. Simultaneous fast measurement of circuit dynamics at multiple sites across the mammalian brain. Nat Methods 13, 325-328, doi:10.1038/nmeth.3770 (2016).
16 Bykov, D. S., Schmidt, O. A., Euser, T. G. & Russell, P. S. J. Flying particle sensors in hollow-core photonic crystal fibre. Nature Photonics 9, 461-U463, doi:10.1038/Nphoton.2015.94 (2015).
17 Cregan, R. F. et al. Single-mode photonic band gap guidance of light in air. Science 285, 1537-1539, doi:DOI 10.1126/science.285.5433.1537 (1999).
18 Russell, P. Photonic crystal fibers. Science 299, 358-362, doi:DOI 10.1126/science.1079280 (2003).
19 Marshall, J. D. et al. Cell-Type-Specific Optical Recording of Membrane Voltage Dynamics in Freely Moving Mice. Cell 167, 1650-1662 e1615, doi:10.1016/j.cell.2016.11.021 (2016).
20 Yotter, R. A. & Wilson, D. M. A review of photodetectors for sensing light-emitting reporters in biological systems. leee Sens J 3, 288-303, doi:10.1109/Jsen.2003.814651 (2003).
21 Brecht, M. & Sakmann, B. Dynamic representation of whisker deflection by synaptic potentials in spiny stellate and pyramidal cells in the barrels and septa of layer 4 rat somatosensory cortex. J Physiol-London 543, 49-70, doi:10.1113/jphysiol.2002.018465 (2002).
22 Haug, H. Brain Sizes, Surfaces, and Neuronal Sizes of the Cortex Cerebri - a Stereological Investigation of Man and His Variability and a Comparison with Some Mammals (Primates, Whales, Marsupials, Insectivores, and One Elephant. Am J Anat 180, 126-142, doi:DOI 10.1002/aja.1001800203 (1987).

## Claims

1. Imaging device (1) configured to detect electromagnetic radiation, which is preferably fluorescence-emission, comprising a sensor device (3) and a collection unit (2), which is configured to collect the electromagnetic radiation and to transmit it to the sensor device (3), wherein the sensor device (3) is a photomultiplier array (3a), preferably a silicon photomultiplier array, and the collection unit (2) comprises a multichannel optical waveguide (4) in the form of a microstructured fiber (5), comprising at least two solid cores (6) separated by at least one hollow tube (7), wherein each solid core (6) is configured to propagate a portion of the collected electromagnetic radiation and wherein the imaging device further comprises a magnification unit (8) which is configured to project the emitted portion of electromagnetic radiation of each individual solid core (6) on one individual pixel (3b) of the photomultiplier array (3a).

2. The imaging device (1) according to claim 1,
**characterized in that**
the multichannel optical waveguide (4) comprises a collection-end-portion (9), which collects the electromagnetic radiation, a main portion (10) and an emission-end-portion (11), where the portions of electromagnetic radiation of each solid core (6) are emitted, wherein the solid cores (6) are arranged in a two dimensional n x m array (12), wherein n and m being natural numbers, wherein the solid cores (6) are in each dimension separated by each other by at least one hollow tube (7), wherein the multichannel optical waveguide (4) has an output in the form of an n x m beam array (12a), wherein n and m being natural numbers.

3. The imaging device (1) according to any one of the previous claims,
**characterized in that**
the solid cores (6) and the hollow tubes (7) consist of a glass material, preferably a silica glass or a polymeric glass or a chalcogenide glass, wherein the solid cores (6) have a diameter in the range of 5 µm to 50 µm, preferably in the range of 8 µm to 10 µm, wherein the hollow tubes (7) have a diameter in the range of 5 µm to 100 µm preferably in the range of 8 µm to 10 µm, wherein the diameter of the multichannel optical waveguide (4) is in the range of 20 µm to 600 µm, preferably in the range between 50 µm to 300 µm, more preferably in the range between 200 µm to 250 µm.

4. The imaging device (1) according to the previous claims 2 to 3,
**characterized in that**
the collection-end-portion (9) of the multichannel optical waveguide (4) comprises hollow tubes (13) which have a larger diameter than the hollow tubes (14) in the main portion (10) of the multichannel optical waveguide (4) and the hollow tubes (15) in the emission-end-portion (11) of the multichannel optical waveguide (4).

5. The imaging device (1) according to the previous claims 2 to 4,
**characterized in that**
the magnification unit (8) comprises a first magnification unit (16) and a second magnification unit (17), wherein both magnification units (16, 17) comprise an infinity corrected optical system in the form of an objective (18, 19) and a tube lens (20, 21), wherein each beam of the beam array (12a) is magnified to about the size of a pixel (3b) of the photomultiplier array (3a).

6. The imaging device (1) according to the preceding claim 5, comprising an optical detector (22) and **characterized in that**
the magnification unit (8) is able to direct a portion of each beam in the beam array (12a) to the optical detector (22), which is able to detect the spatial orientation of the beam array (12a) and is able to provide data regarding spatial orientation of the beam array (12a), wherein the magnification unit (8) comprises a beam splitter (23), by which said portion of each beam in the beam array (12a) is directed to said optical detector (22), wherein said beam splitter (23) is arranged in the optical path between the first magnification unit (16) and the second magnification unit (17).

7. The imaging device (1) according to claim 6,
**characterized in that**
the optical detector (22) is a scientific complementary metal-oxide-semiconductor (sCMOS) camera.

8. The imaging device (1) according to the previous claims 6 to 7,
**characterized in that**
the imaging device (1) comprises a control unit (24), which is able to receive data regarding the spatial orientation of the beam array (12a) from the optical detector (22) and is able to control a multi-axis motorized stage (25) on which the photomultiplier array (3a) is arranged.

9. The imaging device (1) according to claim 8,
**characterized in that**
the control unit (24) is able to process the data regarding spatial orientation of the beam array (12a) and the data from the sensor device (3) and is able to adjust the orientation of the photomultiplier array (3a) by controlling the multi-axis motorized stage (25) in real-time.

10. The imaging device (1) according to the previous claims 6 to 9, **characterized in that**
the imaging device (1) comprises a light source (26), which is coupled in the multi-channel optical waveguide (4) and provides an excitation energy preferably for the fluorescence emission, wherein the excitation energy is controlled by the controlling unit (24), wherein an excitation intensity is controlled based on detected indications on photobleaching in the data provided by the sensor device (3) and / or data provided by the optical detector (22).

11. Imaging system (27) comprising an imaging device (1) according to one of the previous claims and a MRI-scanner (45).

12. A method for analyzing a sample on the basis of fluorescence signals, using an imaging device (1) according to any one of the preceding claims 1-10 comprising:
- collecting the fluorescence signal with the collection unit (2) comprising the multichannel optical waveguide (4) in the form of a microstructured fiber (5), comprising at least two solid cores (6) separated by at least one hollow tube (7), wherein in each solid core (6) propagates a portion of the collected electromagnetic radiation;
- emitting the portion of electromagnetic radiation of each individual solid core (6) in the form of a beam array (12a);
wherein the emitted portion of electromagnetic radiation of each individual solid core (6) is projected on one individual pixel (3b) of the sensor device (3) in the form of a photomultiplier array (3a) by the magnification unit (8).

13. The method according to claim 12,
**characterized in that**.
each beam of the beam array (12a) is magnified by the magnification unit (8) to about the size of an individual pixel (3b) of the photomultiplier array (3a).

14. The method according to the previous claims 12 to 13,
**characterized in that**.
a portion of the beam array (12a) is projected by the magnification unit (8) on an optical detector (22), which is able to detect the spatial orientation of the beam array (12a), wherein the orientation of the photomultiplier array (3a) is adjusted in real-time with a motorized stage (25) according to information regarding the spatial orientation of the beam array (12a).

15. The method according to the previous claims 12 to 14, **characterized in that**
the fluorescence signals are combined with real-time functional magnetic resonance imaging (fMRI imaging), wherein an excitation pattern, for example excitation light frequency, duration and amplitude, can be correlated to a MRI sequence design.

## Patentansprüche

1. Bildgebungsvorrichtung (1), ausgebildet, um elektromagnetische Strahlung zu erkennen, welche bevorzugt Fluoreszenzemission ist, umfassend eine Sensorvorrichtung (3) und eine Erfassungseinheit (2), welche ausgebildet ist, die elektromagnetische Strahlung zu erfassen und diese an die Sensorvorrichtung (3) zu übertragen,
wobei
die Sensorvorrichtung (3) eine Photovervielfacheranordnung (3a), bevorzugt eine Silicium-Photovervielfacheranordnung, ist und wobei die Erfassungseinheit (2) einen mehrkanaligen optischen Wellenleiter (4) in Form einer mikrostrukturierten Faser (5) umfasst, welche mindestens zwei feste Kerne (6) umfasst, welche mittels mindestens einer hohlen Röhre (7) getrennt sind, wobei jeder feste Kern (6) ausgebildet ist, einen Anteil der erfassten elektromagnetischen Strahlung zu verbreiten und wobei die Bildgebungsvorrichtung weiter eine Vergrößerungseinheit (8) umfasst, welche ausgebildet ist, den emittierten Anteil der elektromagnetischen Strahlung jedes einzelnen festen Kernes (6) auf ein einzelnes Pixel (3b) der Photovervielfacheranordnung (3a) zu projizieren.

2. Bildgebungsvorrichtung (1) nach Anspruch 1,
**dadurch gekennzeichnet, dass**
der mehrkanalige optische Wellenleiter (4) einen Erfassungsendanteil (9), welcher die elektromagnetische Strahlung erfasst, einen Hauptanteil (10) und einen Emissionsendanteil (11), in dem die Anteile der elektromagnetischen Strahlung eines jeden festen Kernes (6) emittiert werden, umfasst, wobei die festen Kerne (6) in einer zweidimensionalen n x m-Matrix (12) angeordnet sind, wobei n und m natürliche Zahlen sind, wobei die festen Kerne (6) in jeder Dimension voneinander getrennt sind durch mindestens eine hohle Röhre (7), wobei der mehrkanalige optische Wellenleiter (4) einen Ausgang in Form einer n x m-Strahlmatrix (12a) aufweist, wobei n und m natürliche Zahlen sind.

3. Bildgebungsvorrichtung (1) nach einem der vorhergehenden Ansprüche,
**dadurch gekennzeichnet, dass**
die festen Kerne (6) und die hohlen Röhren (7) aus einem Glasmaterial bestehen, bevorzugt aus einem Quarzglas oder einem Polymerglas oder einem Chalkogenidglas, wobei die festen Kerne (6) einen Durchmesser im Bereich von 5 µm bis 50 µm, bevorzugt im Bereich von 8 µm bis 10 µm, aufweisen, wobei die hohlen Röhren (7) einen Durchmesser im Bereich von 5 µm bis 100 µm, bevorzugt im Bereich von 8 µm bis 10 µm, aufweisen, wobei der Durchmesser des mehrkanaligen optischen Wellenleiters (4) im Bereich von 20 µm bis 600 µm, bevorzugt im Bereich zwischen 50 µm bis 300 µm, weiter bevorzugt im Bereich zwischen 200 µm bis 250 µm, ist.

4. Bildgebungsvorrichtung (1) nach den vorhergehenden Ansprüchen 2 bis 3,
**dadurch gekennzeichnet, dass**
der Erfassungsendanteil (9) des mehrkanaligen optischen Wellenleiters (4) hohle Röhren (13) umfasst, welche einen größeren Durchmesser als die hohlen Röhren (14) im Hauptanteil (10) des mehrkanaligen optischen Wellenleiters (4) und als die hohlen Röhren (15) in dem Emissionsendanteil (11) des mehrkanaligen optischen Wellenleiters (4) aufweisen.

5. Bildgebungsvorrichtung (1) nach den vorhergehenden Ansprüchen 2 bis 4,
**dadurch gekennzeichnet, dass**
die Vergrößerungseinheit (8) eine erste Vergrößerungseinheit (16) und eine zweite Vergrößerungseinheit (17) umfasst, wobei beide Vergrößerungseinheiten (16, 17) ein unendlich-korrigiertes optisches System in Form eines Objektives (18, 19) und einer Tubuslinse (20, 21) umfassen, wobei jeder Strahl der Strahlmatrix (12a) etwa auf die Größe eines Pixels (3b) der Photovervielfacheranordnung (3a) vergrößert wird.

6. Bildgebungsvorrichtung (1) nach dem vorhergehenden Anspruch 5, umfassend einen optischen Detektor (22) und
**dadurch gekennzeichnet, dass**
die Vergrößerungseinheit (8) geeignet ist, einen Anteil eines jeden Strahles in der Strahlmatrix (12a) an den optischen Detektor (22) zu leiten, welcher geeignet ist, die räumliche Orientierung der Strahlmatrix (12a) zu erkennen und geeignet ist, Daten hinsichtlich der räumlichen Orientierung der Strahlmatrix (12a) bereitzustellen, wobei die Vergrößerungseinheit (8) einen Strahlteiler (23) aufweist, durch welchen der Anteil eines jeden Strahles in der Strahlmatrix (12a) an den optischen Detektor (22) geleitet wird, wobei der Strahlteiler (23) in dem optischen Pfad zwischen der ersten Vergrößerungseinheit (16) und der zweiten Vergrößerungseinheit (17) angeordnet ist.

7. Bildgebungsvorrichtung (1) nach Anspruch 6,
**dadurch gekennzeichnet, dass**
der optische Detektor (22) eine wissenschaftlich komplementäre Metall-Oxid-Halbleiter (sCMOS) Kamera ist.

8. Bildgebungsvorrichtung (1) nach den vorhergehenden Ansprüchen 6 bis 7,
**dadurch gekennzeichnet, dass**
die Bildgebungsvorrichtung (1) eine Steuereinheit (24) umfasst, welche geeignet ist, Daten hinsichtlich der räumlichen Orientierung der Strahlmatrix (12a) von dem optischen Detektor (22) zu empfangen, und geeignet ist, einen mehrachsigen motorisierten Tisch (25) zu steuern, auf welcher die Photovervielfacheranordnung (3a) angeordnet ist.

9. Bildgebungsvorrichtung (1) nach Anspruch 8,
**dadurch gekennzeichnet, dass**
die Steuereinheit (24) geeignet ist, die Daten hinsichtlich der räumlichen Orientierung der Strahlmatrix (12a) und die Daten von der Sensorvorrichtung (3) zu verarbeiten und geeignet ist, die Orientierung der Photovervielfacheranordnung (3a) durch Steuern des mehrachsigen motorisierten Tisches (25) in Echtzeit anzupassen.

10. Bildgebungsvorrichtung (1) nach den vorhergehenden Ansprüchen 6 bis 9,
**dadurch gekennzeichnet, dass**
die Bildgebungsvorrichtung (1) eine Lichtquelle (26) umfasst, welche in dem mehrkanaligen optischen Wellenleiter (4) gekoppelt ist und eine Anregungsenergie, bevorzugt für die Fluoreszenzemission, bereitstellt, wobei die Anregungsenergie mittels der Steuereinheit (24) gesteuert ist, wobei eine Anregungsintensität basierend auf detektierten Anzeichen von Photobleichung in den mittels der Sensorvorrichtung (3) bereitgestellten Daten und/oder mittels des optischen Detektors (22) bereitgestellten Daten gesteuert ist.

11. Bildgebungssystem (27) umfassend eine Bildgebungsvorrichtung (1) nach einem der vorhergehenden Ansprüche und einen MRI-Scanner (45).

12. Verfahren zum Auswerten einer Probe basierend auf Fluoreszenzsignalen, unter Verwendung einer Bildgebungsvorrichtung (1) nach einem der vorhergehenden Ansprüche 1-10, umfassend:
- Erfassen des Fluoreszenzsignals mit der Erfassungseinheit (2) umfassend den mehrkanaligen optischen Wellenleiter (4) in Form einer mikrostrukturierten Faser (5), umfassend mindestens zwei feste Kerne (6), welche durch mindestens eine hohle Röhre (7) voneinander getrennt sind, wobei sich in jedem festen Kern (6) ein Anteil der gesammelten elektromagnetischen Strahlung verbreitet;
- Emittieren des Anteils der elektromagnetischen Strahlung jedes einzelnen festen Kernes (6) in Form einer Strahlmatrix (12a);
wobei der emittierte Anteil der elektromagnetischen Strahlung eines jeden einzelnen festen Kernes (6) auf ein einzelnes Pixel (3b) der Sensorvorrichtung (3) in Form einer Photovervielfacheranordnung (3a) mittels der Vergrößerungseinheit (8) projiziert wird.

13. Verfahren nach Anspruch 12,
**dadurch gekennzeichnet, dass**
jeder Strahl der Strahlmatrix (12a) mittels der Vergrößerungseinheit (8) auf etwa die Größe eines einzelnen Pixels (3b) der Photovervielfacheranordnung (3a) vergrößert wird.

14. Verfahren nach den vorhergehenden Ansprüchen 12 bis 13,
**dadurch gekennzeichnet, dass**
ein Anteil der Strahlmatrix (12a) mittels der Vergrößerungseinheit (8) auf einen optischen Detektor (22) projiziert wird, welcher geeignet ist, die räumliche Orientierung der Strahlmatrix (12a) zu erkennen, wobei die Orientierung der Photovervielfacheranordnung (3a) in Echtzeit mit einem motorisierten Tisch (25) angepasst wird gemäß Informationen hinsichtlich der räumlichen Orientierung der Strahlmatrix (12a).

15. Verfahren nach den vorhergehenden Ansprüchen 12 bis 14,
**dadurch gekennzeichnet, dass**
die Fluoreszenzsignale kombiniert sind mit funktioneller Magnetresonanztomographie (fMRI-Bildgebung) in Echtzeit, wobei ein Anregungsmuster, beispielsweise Anregungslichtfrequenz, -dauer und -amplitude, mit einer MRI-Sequenzgestaltung korreliert werden kann.

## Revendications

1. Dispositif d'imagerie (1) configuré pour détecter un rayonnement électromagnétique, qui est de préférence à émission de fluorescence, comprenant un dispositif de capteur (3) et une unité de collecte (2), qui est configurée pour collecter le rayonnement électromagnétique et pour le transmettre au dispositif de capteur (3), le dispositif de capteur (3) étant un réseau de photomultiplicateurs (3a), de préférence un réseau de photomultiplicateurs en silicium, et l'unité de collecte (2) comprenant un guide d'onde optique multicanaux (4) sous la forme d'une fibre microstructurée (5), comprenant au moins deux noyaux pleins (6) séparés par au moins un tube creux (7), chaque noyau plein (6) étant configuré pour propager une partie du rayonnement électromagnétique collecté, et le dispositif d'imagerie comprenant en outre une unité de grossissement (8) qui est configurée pour projeter la partie émise de rayonnement électromagnétique de chaque noyau plein individuel (6) sur un pixel individuel (3b) du réseau de photomultiplicateurs (3a).

2. Dispositif d'imagerie (1) selon la revendication 1,
**caractérisé par le fait que**
le guide d'onde optique multicanaux (4) comprend une partie d'extrémité de collecte (9) qui collecte le rayonnement électromagnétique, une partie principale (10) et une partie d'extrémité d'émission (11), d'où les parties de rayonnement électromagnétique de chaque noyau plein (6) sont émises, les noyaux pleins (6) étant disposés dans un réseau n x m en deux dimensions (12), n et m étant des entiers naturels, les noyaux pleins (6) étant dans chaque dimension séparés l'un de l'autre par au moins un tube creux (7), le guide d'onde optique multicanaux (4) ayant une sortie sous la forme d'un réseau de faisceaux n x m (12a), n et m étant des entiers naturels.

3. Dispositif d'imagerie (1) selon l'une quelconque des revendications précédentes,
**caractérisé par le fait que**
les noyaux pleins (6) et les tubes creux (7) sont constitués d'un matériau en verre, de préférence un verre de silice ou un verre polymère ou un verre de chalcogénure, les noyaux pleins (6) ayant un diamètre dans la plage allant de 5 µm à 50 µm, de préférence dans la plage allant de 8 µm à 10 µm, les tubes creux (7) ayant un diamètre dans la plage allant de 5 µm à 100 µm, de préférence dans la plage allant de 8 µm à 10 µm, le diamètre du guide d'onde optique multicanaux (4) étant dans la plage allant de 20 µm à 600 µm, de préférence dans la plage allant de parmi 50 µm à 300 µm, de façon davantage préférée dans la plage allant de parmi 200 µm à 250 µm.

4. Dispositif d'imagerie (1) selon les revendications précédentes 2 à 3,
**caractérisé par le fait que**
la partie d'extrémité de collecte (9) du guide d'onde optique multicanaux (4) comprend des tubes creux (13) qui ont un diamètre plus grand que les tubes creux (14) dans la partie principale (10) du guide d'onde optique multicanaux (4) et les tubes creux (15) dans la partie d'extrémité d'émission (11) du guide d'onde optique multicanaux (4).

5. Dispositif d'imagerie (1) selon les revendications précédentes 2 à 4,
**caractérisé par le fait que**
l'unité de grossissement (8) comprend une première unité de grossissement (16) et une seconde unité de grossissement (17), les deux unités de grossissement (16, 17) comprenant un système optique corrigé à l'infini sous la forme d'un objectif (18, 19) et d'une lentille de tube (20, 21), chaque faisceau du réseau de faisceaux (12a) étant agrandi jusqu'à environ la taille d'un pixel (3b) du réseau de photomultiplicateurs (3a).

6. Dispositif d'imagerie (1) selon la revendication précédent 5, comprenant un détecteur optique (22), et
**caractérisé par le fait que**
l'unité de grossissement (8) est apte à diriger une partie de chaque faisceau dans le réseau de faisceaux (12a) vers le détecteur optique (22), qui est apte à détecter l'orientation spatiale du réseau de faisceaux (12a) et est apte à fournir des données relatives à l'orientation spatiale du réseau de faisceaux (12a), l'unité de grossissement (8) comprenant un séparateur de faisceau (23), par lequel ladite partie de chaque faisceau dans le réseau de faisceaux (12a) est dirigée vers ledit détecteur optique (22), ledit séparateur de faisceau (23) étant disposé dans le trajet optique entre la première unité de grossissement (16) et la seconde unité de grossissement (17).

7. Dispositif d'imagerie (1) selon la revendication 6,
**caractérisé par le fait que**
le détecteur optique (22) est une caméra scientifique à semi-conducteur complémentaire à l'oxyde de métal (sCMOS).

8. Dispositif d'imagerie (1) selon les revendications précédentes 6 à 7,
**caractérisé par le fait que**
le dispositif d'imagerie (1) comprend une unité de commande (24), qui est apte à recevoir des données relatives à l'orientation spatiale du réseau de faisceaux (12a) provenant du détecteur optique (22) et est apte à commander une platine motorisée à axes multiples (25) sur laquelle le réseau de photomultiplicateurs (3a) est disposé.

9. Dispositif d'imagerie (1) selon la revendication 8,
**caractérisé par le fait que**
l'unité de commande (24) est apte à traiter les données relatives à l'orientation spatiale du réseau de faisceaux (12a) et les données provenant du dispositif de capteur (3) et est apte à ajuster l'orientation du réseau de photomultiplicateurs (3a) par commande de la platine motorisée à axes multiples (25) en temps réel.

10. Dispositif d'imagerie (1) selon les revendications précédentes 6 à 9,
**caractérisé par le fait que**
le dispositif d'imagerie (1) comprend une source de lumière (26), qui est couplée dans le guide d'onde optique multicanaux (4) et fournit une énergie d'excitation de préférence pour l'émission de fluorescence, l'énergie d'excitation étant commandée par l'unité de commande (24), une intensité d'excitation étant commandée sur la base d'indications détectées de photoblanchiment dans les données fournies par le dispositif de capteur (3) et/ou des données fournies par le détecteur optique (22).

11. Système d'imagerie (27) comprenant un dispositif d'imagerie (1) selon l'une des revendications précédentes et un scanner IRM (45).

12. Procédé pour analyser un échantillon sur la base de signaux de fluorescence, à l'aide d'un dispositif d'imagerie (1) selon l'une quelconque des revendications 1-10, comprenant :
- collecter le signal de fluorescence avec l'unité de collecte (2) comprenant le guide d'onde optique multicanaux (4) sous la forme d'une fibre microstructurée (5), comprenant au moins deux noyaux pleins (6) séparés par au moins un tube creux (7), dans chaque noyau plein (6) se propageant une partie du rayonnement électromagnétique collecté ;
- émettre la partie de rayonnement électromagnétique de chaque noyau plein individuel (6) sous la forme d'un réseau de faisceaux (12a) ;
la partie émise de rayonnement électromagnétique de chaque noyau plein individuel (6) étant projetée sur un pixel individuel (3b) du dispositif de capteur (3) sous la forme d'un réseau de photomultiplicateurs (3a) par l'unité de grossissement (8).

13. Procédé selon la revendication 12,
**caractérisé par le fait que**
chaque faisceau du réseau de faisceaux (12a) est agrandi par l'unité de grossissement (8) jusqu'à environ la taille d'un pixel individuel (3b) du réseau de photomultiplicateurs (3a).

14. Procédé selon les revendications précédentes 12 à 13,
**caractérisé par le fait qu'**
une partie du réseau de faisceaux (12a) est projetée par l'unité de grossissement (8) sur un détecteur optique (22), qui est apte à détecter l'orientation spatiale du réseau de faisceaux (12a), l'orientation du réseau de photomultiplicateurs (3a) étant ajustée en temps réel avec une platine motorisée (25) selon des informations relatives à l'orientation spatiale du réseau de faisceaux (12a).

15. Procédé selon les revendications précédentes 12 à 14,
**caractérisé par le fait que**
les signaux de fluorescence sont combinés à une imagerie par résonance magnétique fonctionnelle (imagerie IRMf) en temps réel, un motif d'excitation, par exemple une fréquence, une durée et une amplitude de lumière d'excitation, pouvant être mis en corrélation avec un modèle de séquence IRM.
